# EUROPEAN PATENT APPLICATION

(11) **EP 4 162 907 A1**
(43) Date of publication of application: **12.04.2023**
(21) Application number: 22164813.2
(22) Date of filing: 28.03.2022
(51) Int. Cl.: A61F 13/15, A61F 13/551, A61F 13/56, B65D 85/00, B65D 65/42, D21H 27/10

(54) **WRAPPER FOR ABSORBENT ARTICLE**

(30) Priority: 08.10.2021 EP 21201775
(71) Applicant: Ontex BV, 9255 Buggenhout (BE); Ontex Group NV, 9320 Erembodegem (BE)
(72) Inventor: Descheemaecker, Evan, 9930 Zomergem (BE); VAN DAMME, Claudine, 9900 Eeklo (BE); Vandenbossche, Jeroen, 9230 Wetteren (BE); Wagner, Christoph, D-56727 Mayen (DE)
(74) Representative: Saurat, Thibault

(57) **Abstract**

The invention pertains to a wrapper (22) for an absorbent article (10) comprising a release paper (51,51') comprising a layer of cellulosic fibers (33), a layer of peeling agent (32) arranged onto the layer of cellulosic fibers (33). According to the invention, the layer of cellulosic fibers (33) comprises a basis weight comprised between 10 gsm and 40 gsm. The invention also relates to an assembly comprising an absorbent article (10) and such wrapper (22) and a process to manufacture such assembly (10).

## Description

### TECHNICAL FIELD

The disclosure pertains to the technical field of absorbent articles for hygiene products and the packaging of such absorbent articles. In particular, the present disclosure relates to individually packaged absorbent articles selected from sanitary napkins, adult incontinence pads or panty liners, which are configured to collect and contain blood, menses, urine, vaginal fluids and avoid leakage. The present disclosure also pertains to the packaging process to wrap or enclose such absorbent articles.

### BACKGROUND

Absorbent articles selected from sanitary napkins, adult incontinence pads or pantyliners, used to collect vaginal discharges are well known in the art. Typically, these absorbent articles commonly comprise a topsheet, a backsheet and an absorbent core arranged in-between said topsheet and backsheet and are typically individually enclosed, or packaged, in a wrapper also commonly called a pouch. These wrappers or pouches are made out of plastic films such as a polypropylene or polyethylene films since these material have several beneficial physical properties such as flexibility, deformability, *etc.* as well as for cost reasons. However, these wrappers are always made from petroleumderived materials and thus are not environment-friendly.

There is still a need to improve the environmental impact of these wrappers and offer a solution that involves less plastic without degrading the performance of the packaging of these absorbent articles.

The invention thereto aims to provide an environment-friendly packaging for absorbent articles selected from sanitary napkins, adult incontinence pads or pantyliners.

### SUMMARY OF THE INVENTION

The invention relates to a wrapper for an absorbent article comprising a release paper, said wrapper comprising a layer of cellulosic fibers wherein the layer of cellulosic fibers comprises a basis weight comprised between 10 gsm and 40 gsm.

According to an embodiment, the layer of cellulosic fibers consists essentially of paper material.

According to an embodiment, the layer of cellulosic fibers comprises a basis weight comprised between 15 gsm and 35 gsm, more preferably between 16 gsm and 30 gsm, more preferably between 18 gsm and 29 gsm, more preferably between 20 gsm and 28 gsm.

By using a cellulose pulp such as paper or other natural fibers corresponding to environment friendly materials, the environmental impact of the packaging is lowered. By comprising a basis weight comprised between 10 gsm and 40 gsm, the packaging layer retains the functionality of the plastic film conventionally used. The basis weight is sufficiently high enough to ensure that the wrapper is robust to contain an absorbent article and not tear easily. The basis weight is sufficiently low enough to ensure that the wrapper is flexible and can be easily folded. A wrapper comprising a layer of cellulosic fiber comprising a basis weight between 18 gsm and 29 gsm, or more preferably between 20 gsm and 28 gsm, is well balanced and displays a good compromise between robustness and flexibility. The basis weight of the layer of cellulosic fibers is preferably measured according to the ISO 536 standard or any standard method.

According to an embodiment, the wrapper further comprises a layer of sealing agent arranged onto the layer of cellulosic fibers.

According to an embodiment, the layer of cellulosic fibers comprises cellulosic fibers with an average fiber length comprised between 0,5 and 10 mm, preferably between 0,7 and 5 mm, more preferably between 1 and 4 mm and/or with an average fiber diameter comprised between 0,5 and 50 µm.

According to an embodiment, the layer of cellulosic fibers comprises a thickness comprised between 5 µm and 150 µm, preferably between 20 µm and 80 µm, more preferably between 30 µm and 60 µm.

Average fiber length or average fiber diameter can be determined by using any standard image processing analysis method such as measuring said fiber length and/or fiber diameter with a microscope or a similar apparatus and extracting said data (average fiber length, average fiber diameter) from the images.

According to an embodiment, the layer of cellulosic fibers comprises a first sub-layer of cellulosic fibers and a second layer of cellulosic fibers, the cellulosic fibers in the second sub-layer having a greater average fiber length than the first sub-layer.

According to an embodiment, said wrapper further comprises a layer of peeling agent comprising a peeling agent selected from silicon resin series, fluororesin series, octadecilisocyanate series, said layer of peeling agent comprising a basis weight comprised between 0,3 gsm and 3 gsm, preferably between 0,5 gsm and 1 gsm.

According to an embodiment, the layer of sealing agent has a basis weight comprised between 1 gsm and 20 gsm, preferably between 3 gsm and 15 gsm, more preferably between 5 gsm and 10 gsm.

According to an embodiment, the wrapper comprises a rectangular configuration, the sealing agent being arranged at least on the periphery of the wrapper along at least two edges arranged on opposite sides wherein the wrapper further comprising uncoated portions arranged at least at one of the corners of the rectangular configuration of the wrapper. The wrapper comprises a total surface area greater than the absorbent article, as seen from a top view, meaning that the wrapper has a length and/or width greater than those of the absorbent article.

The invention also pertains to an assembly comprising a wrapper as described previously and an absorbent article, the absorbent article comprising a topsheet, a backsheet and an absorbent core arranged in-between wherein the absorbent article comprises a first adhesive layer and a release paper, preferably the first adhesive layer is arranged in-between the backsheet and the release paper.

According to an embodiment, the absorbent article comprises wings and a release paper.

According to an embodiment, the absorbent article comprises a second adhesive layer arranged in-between the wrapper and the release paper.

According to an embodiment, the first adhesive layer comprises a basis weight that is greater than the basis weight of the second adhesive layer, preferably at least 1.2 time greater, more preferably at least 5 times greater.

The invention also pertains to a process to manufacture an assembly as described above, the process comprising the following steps:
- providing at least one layer of cellulosic fibers ;
- optionally applying a layer of sealing agent on a surface of said at least one layer of cellulosic fibers ;
- applying an absorbent article onto said layer of cellulosic fibers or layer of sealing agent in a way that the release paper is arranged between the backsheet and the wrapper ;
- folding the wrapper and the absorbent article altogether to enclose the absorbent article within the wrapper; and
- applying heat and/or pressure on the wrapper to seal the absorbent article within the wrapper.

According to an embodiment, the process comprises an additional step of printing a graphic element on said wrapper.

According to an embodiment, the process comprises an additional step of applying a second layer of adhesive on the wrapper prior to applying the light incontinence pad.

A present embodiment relates to a wrapper for an absorbent article comprising a layer of cellulosic fibers, a layer of peeling agent arranged onto the layer of cellulosic fibers wherein the layer of cellulosic fibers comprises a basis weight comprised between 10 gsm and 40 gsm. The layer of peeling agent is preferably arranged on an inner surface of the wrapper that is configured to come into contact with an absorbent article.

According to an embodiment, the wrapper further comprises a layer of sealing agent arranged onto the layer of cellulosic fibers.

According to an embodiment, the wrapper comprises a closing tape arranged on an outer surface of the wrapper, said closing tape comprising a grasping section free of adhesive and an attaching section that comprises adhesive so that the closing tape can adhere to the outer surface of the wrapper.

According to an embodiment, the wrapper comprises a rectangular configuration, the sealing agent being arranged on the periphery of the wrapper along at least two edges arranged on opposite sides thereby defining a frame with an inner area, the peeling agent being arranged within the inner area of the frame defined by the sealing agent.

According to an embodiment, the wrapper comprises uncoated portions arranged at least at one of the corners of the rectangular configuration of the wrapper.

According to an embodiment, the closing tape is arranged on the first sub-layer of cellulosic fibers and the pealing agent and sealing agent are arranged on the second layer of cellulosic fibers.

All of these embodiments mentioned above can be taken individually or in combination.

The invention also pertains to an assembly comprising a wrapper according to as described above and an absorbent article, the absorbent article comprising a topsheet, a backsheet and an absorbent core arranged in-between wherein the absorbent article comprises an adhesive layer, the adhesive layer being arranged between the backsheet and the layer or peeling agent of the wrapper. The invention also relates to a process to manufacture an assembly as described above, the process comprising the following steps:
a) providing at least one layer of cellulosic fibers;
b) applying a layer of peeling agent on a surface of said at least one layer of cellulosic fibers;
c) optionally applying a layer of sealing agent (34) on a surface of said at least one layer of cellulosic fibers (33);
d) applying an absorbent article on said layer of peeling agent in a way that the adhesive layer being arranged between the backsheet and the layer of peeling agent;
e) folding the wrapper and the absorbent article altogether to enclose the absorbent article within the wrapper; and
f) applying heat and/or pressure on the wrapper to seal the absorbent article within the wrapper;
g) optionally applying a closing tape on the outer surface of wrapper.

According to an embodiment, the process comprises an additional step of printing a graphic element on said wrapper.

All of these embodiments mentioned above can be taken individually or in combination.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG. 1** illustrates a diagrammatic cross section of an absorbent article comprising a wrapper according to an embodiment of the invention.
**FIG. 2** shows a schematic top view of according to an embodiment
**FIG. 3** illustrates a perspective view of a wrapper enclosing an absorbent article according to an embodiment.
**FIG. 4** illustrates a schematic top view of a wrapper according to an embodiment.
**FIG. 5** illustrates a schematic top view of a wrapper according to an embodiment.
**FIG. 6** illustrates a schematic top view of a wrapper according to an embodiment.
**FIG. 7** illustrates a diagrammatic cross section of an absorbent article comprising a wrapper according to an embodiment of the invention.
**FIG. 8** illustrates an assembly comprising an absorbent article and a wrapper in an exploded view according to an embodiment of the invention.
**FIG. 9** illustrates a schematic top view of an absorbent article according to an embodiment.

### DETAILED DESCRIPTION OF THE INVENTION

The present invention concerns the packaging of individually packaged absorbent articles selected from sanitary napkins, adult incontinence pads or pantyliners.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The recitation of numerical ranges by endpoints includes all numbers and fractions subsumed within that range, as well as the recited endpoints.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

"Absorbent article" refers to devices that absorb and contain liquid, and more specifically, refers to devices that are placed against or in proximity to the body of the wearer to absorb and contain the various exudates discharged from the body. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious topsheet, a backsheet joined to the topsheet, and an absorbent core positioned and held between the topsheet and the backsheet. The topsheet is operatively permeable to the liquids that are intended to be held or stored by the absorbent article, and the backsheet may or may not be substantially impervious or otherwise operatively impermeable to the intended liquids. The absorbent article may also include other components, such as acquisition distribution layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

"Front", "rear or back", and "crotch" end of the absorbent article as used herein typically refer for the "front" end of the article is that which is most proximal to the front of the subject when worn, the "rear or back" end of the article is that which is most proximal to the rear or back of the subject when worn, and the "crotch" portion of the core is the middle portion of the absorbent article between the "front" and "rear or back" portions.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition distribution layer", "ADL" or "surge management portion" refers to a sub-layer which preferably is a nonwoven wicking layer arranged directly under the topsheet of an absorbent product, which speeds up the transport and improves distribution of fluids throughout the absorbent core. The surge management portion has the ability to quickly collect and temporarily hold liquid surges, and to transport the liquid from its initial entrance point to other parts of the absorbent structure, particularly the retention portion. This configuration can help prevent the exudates from pooling and collecting on the portion of the absorbent garment positioned against the wearer's skin.

The term "adhesive" as used herein is intended to refer to any suitable hot melt, water or solvent borne adhesive that can be applied to a surface of a film layer in the required pattern or network of adhesive areas to form the film-nonwoven laminate of the present disclosure. Accordingly, suitable adhesives include conventional hot melt adhesives, pressure-sensitive adhesives and reactive adhesives (i.e., polyurethane). The absorbent article can comprise different kinds of adhesive. For example, the absorbent article can comprise hot melt adhesive for bonding the topsheet to the backsheet and non-structural adhesive for bonding the absorbent article to the wrapper.

As used herein, the term "non-structural adhesive" refers to any adhesive with a low bonding strength usually demonstrating a load-carrying capacity of less than 1000 pound-force per square inch (psi). Such non-structural adhesives comprise for example vinyls, polychloropene and polyurethane.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which topsheet is directly joined to backsheet by affixing topsheet directly to backsheet, and configurations wherein topsheet is joined to backsheet by affixing topsheet to intermediate members which in turn are affixed to backsheet. Topsheet and backsheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet to backsheet. It should be readily appreciated that the above-described attachment means may also be employed to interconnect and assemble together the various other component parts of the article described herein.

The term "backsheet" or "backsheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing there through. Examples of breathable backsheet materials are porous polymeric films, nonwoven laminates of spunbond and meltblown layers and laminates of porous polymeric films and nonwoven materials.

As used herein, the term "basis weight" refers to an average weight, or a range of weight, of the component in question, namely the layer of cellulosic fibers or the layer of peeling agent, per square meter. Term such as "grammage" is equivalent to "basis weight" and is expressed in grams per square meter, g/m² or gsm.

For instance, the basis weight of the layer of cellulosic fibers is preferably measured according to the ISO 536 standard or any standard method. The amount of peeling agent or sealing agent on the wrapper can be measured by taking a sample of the wrapper where the peeling agent or the sealing agent is arranged, weighing said sample before and after removal of the peeling agent or sealing agent (in grams) the difference giving the weight of the peeling agent or sealing agent, and dividing the weight of peeling agent or sealing agent by the area of the sample (in m2) thereby obtaining the amount of peeling agent or sealing agent in gsm.

As used herein, the term "body-facing", "skin-facing" or "bodyside" side or surface means that surface of the article or component which is intended to be disposed toward or placed adjacent to the body of the wearer during ordinary use, while the "outward", "outward-facing" or "garment-facing" side or surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

As used herein, the term "cellulosic" or "cellulosic fibers" is meant to include any material having cellulose as a major constituent, and specifically comprising at least 50 percent by weight cellulose or a cellulose derivative. Thus, the term includes cotton, typical wood pulps, nonwoody cellulosic fibers, kraft paper, cellulose acetate, cellulose triacetate, rayon, thermomechanical wood pulp, chemical wood pulp, debonded chemical wood pulp, milkweed, or bacterial cellulose.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "garment" means any type of apparel which may be worn. This includes diapers, training pants, incontinence products, surgical gowns, industrial workwear and coveralls, undergarments, pants, shirts, jackets and the like.

Many of the known superabsorbent polymer particles exhibit gel blocking. "Gel blocking" occurs when superabsorbent polymer particles are wetted and the particles swell so as to inhibit fluid transmission to other regions of the absorbent structure. Wetting of these other regions of the absorbent member therefore takes place via a very slow diffusion process. In practical terms, this means acquisition of fluids by the absorbent structure is much slower than the rate at which fluids are discharged, especially in gush situations. Leakage from the absorbent article can take place well before the particles of SAP in the absorbent member are even close to being fully saturated or before the fluid can diffuse or wick past the "blocking" particles into the rest of the absorbent member. Gel blocking can be a particularly acute problem if the superabsorbent polymer particles do not have adequate gel strength and deform or spread under stress once the particles swell with absorbed fluid.

The term "graphic" or "graphic element" includes, but is not limited to, any type of design, image, mark, figure, codes, words, patterns, or the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layer or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood. The material is typically lightweight and has absorbent properties.

By "channels", it is meant that the structure referred to (e.g. the absorbent core) comprises recessed regions forming visible conduits or passages typically extending along the longitudinal axis of the core and having a depth in a direction perpendicular to said longitudinal axis. By "visible" it is herein intended clearly visible by naked eye and typically that the channels have a width generally greater than 1mm, preferably from 5mm to 50 mm, more preferably from 8mm to 40mm, more preferably from 10mm to 30mm, even more preferably from greater than 10mm to less than 25mm.

By "release paper" it is meant a peelable piece of paper that is arranged and configured to be peeled off, or separated from, an adhesive layer. The release paper is preferably formed of a paper material such as woodfree paper or glassine paper, having a releasing layer, or releasing coating, of silicon-based resin thereby ensuring that the release paper can be peeled off an adhesive material. In other words, a "release paper" refers herein to a sheet of paper comprising one or more layers, for example a layer of cellulosic fibers with a layer, or coating, of peeling agent, that is arranged to cover at least partly, preferably entirely, a layer of adhesive and that can be easily peeled off from said layer of adhesive.

"Strip(s)" and "stripe(s)" are used herein as equivalent term with no real distinction and refer to a long narrow area of material.

By "substantially", it is meant at least the majority of the structure referred to.

Use of the term "substrate" includes, but is not limited to, woven or nonwoven webs, porous films, ink permeable films, cellulosic pulp, paper, composite structures, or the like.

Superabsorbent materials suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCI). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, the particle size is typically ranging between 100 to 800 µm, preferably between 300 to 600 µm, more preferably between 400 to 500 µm.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. The topsheet fabrics may be composed of a substantially hydrophobic material, and the hydrophobic material may optionally be treated with a surfactant or otherwise processed to impart a desired level of wettability and hydrophilicity.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

By the term "wrapper" as used herein, is meant a packaging component that enclose the absorbent article prior to use. Terms such as "pouch", "wrapping sheet", "packaging sheet" or "wrapping construction" are equivalent to "wrapper".

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

Embodiments according to the disclosure will now be described. It is understood that technical features described in one or more embodiments may be combined with one or more other embodiments without departing from the intention of the disclosure and without generalization therefrom.

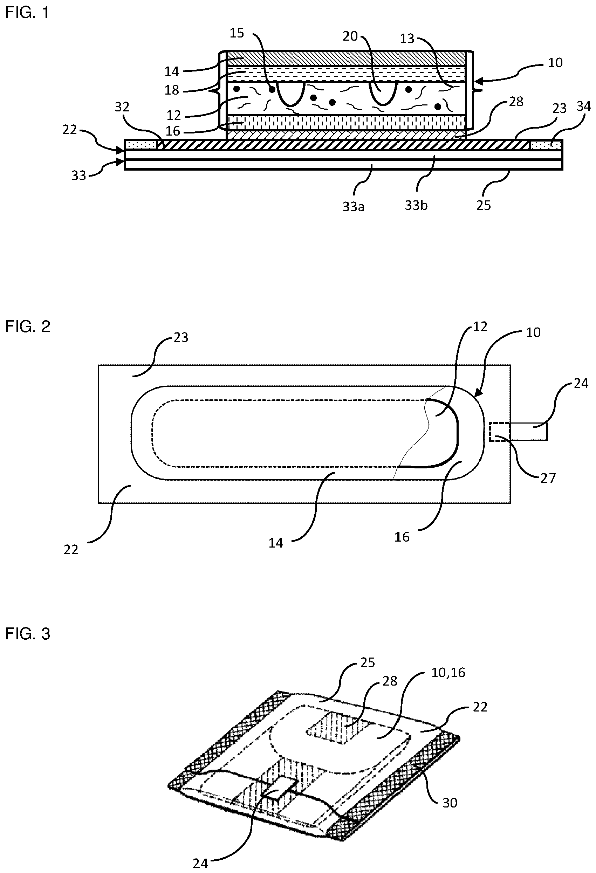

The embodiments illustrated in FIG. 1 to FIG. 9 show different types of absorbent article such as sanitary napkins or light incontinence pads. These absorbent articles 10,10' are used to collect absorb, distribute and store body exudates, for example, a sanitary napkin 10 is adapted for collecting menses or menstrual blood whereas a light incontinence pad 10' is adapted for collecting urine. Hence, each type of absorbent article presents different properties such as size, amount of absorbent material, amount of adhesive to stay fastened to the undergarment since they have different purposes (menstruation, incontinence,...). The wrapper for one type of absorbent article can have at least one feature in common with the wrapper for another type of absorbent article and still present different properties such as basis weight, graphic elements, *etc.,* or in other words a feature present in a wrapper for one type of absorbent article can be present in a wrapper for another type of absorbent article.

As illustrated in Fig.1 and Fig.2, a disposable absorbent article 10, for example a sanitary napkin, according to the present disclosure comprises an absorbent core 12 arranged between a liquid permeable topsheet 14 and a liquid impermeable backsheet 16. An optional acquisition distribution layer 18, commonly called ADL, can be positioned between said topsheet 14 and said absorbent core 12. The absorbent core 12 comprises absorbent material selected from the group consisting of cellulose fibers 13 such as fluff pulp, superabsorbent polymers 15 or a combination thereof. The absorbent core 12 can comprise at least one channel 20 substantially free of cellulose fibers and/or substantially free of superabsorbent polymer (as illustrated in Fig.1). The backsheet 16 is joined to the topsheet 14 with the absorbent core 12, and the optional ADL 18, being positioned and held between the topsheet 14 and the backsheet 16. As illustrated in Fig. 1, the topsheet 14 and the backseet 16 are associated to one another. The topsheet 14 is arranged on the body-facing side or surface whereas the backsheet 16 is arranged on the garment-facing side or surface.

As illustrated in FIG. 3, the absorbent article 10 is enclosed, here in a folded configuration, in a wrapper 22 or pouch. The wrapper 22 acts as a barrier ensuring that the unused absorbent article 10 is protected against contamination prior to use. The wrapper 22 can also be used as a disposal-wrap around a soiled absorbent article to be disposed of.

As shown in FIG. 2, the wrapper 22 has a generally rectangular configuration with an inner surface 23 on which the absorbent article 10 is arranged and an outer surface 25 on which a closing tape 24 is arranged. In other terms, the inner surface 23 is arranged on the body-facing side and the outer surface is arranged on the garment-facing side. A closing tape 24 for sealing the wrapping construction and help maintaining the wrapper 22 in a closed configuration as illustrated in Fig.3, is provided at a front end 36 portion of the wrapper 22. The closing tape 24 comprises a grasping section 26 that is free of adhesive so that the user can grasp the closing tape 24 and open up the wrapper 22 to release the absorbent article 10 from said wrapper. The closing tape 24 also comprises an attaching section 27 that comprises adhesive so that the closing tape 24 can adhere to the outer surface 25 of the wrapper 22 especially when it is used as a disposal wrap. When used as a disposal-wrap, the closing tape 24 can be used to contain the soiled article within the wrapper 22.

The wrapper 22 according to the invention comprises cellulosic fibers or in other terms, the wrapper 22 contains a sustainable material and more specifically cellulose pulp. Preferably, the wrapper 22 comprises paper material, or cellulosic fibres, coming from natural sources for example and not limited to wood (softwood or hardwood), cotton or hemp. A nonwoven fabric of cellulosic fibers, e.g. paper, is less flexible and rougher than a plastic film. To that intent, the wrapper 22 comprises a layer consisting essentially of cellulosic fibers 33, *e.g.* a paper layer, comprising a basis weight comprised between 10 gsm and 40 gsm, preferably between 15 gsm and 35 gsm, more preferably between 20 gsm and 30 gsm. More preferably, the layer of cellulosic faber 33 of the wrapper 22 consists essentially of paper material. By using a pulp such as paper or any other natural cellulose fibers corresponding to an environment friendly material, the environmental impact of the packaging is lowered. By comprising a basis weight comprised between 10 gsm and 40 gsm, preferably between 18 gsm and 29 gsm, the pouch 22 retains the functionalities of the plastic film conventionally used. The basis weight is sufficiently high to ensure that the wrapper 22 is robust enough to contain an absorbent article 10 and not tear easily. The basis weight is sufficiently low to ensure that the wrapper is flexible enough and can be easily folded. Another parameter of the layer of cellulosic fibers 33 is its thickness, specifically, the layer of cellulosic fibers 33 has a thickness between 5 µm and 150 µm, preferably between 20 µm and 80 µm, more preferably between 30 µm and 60 µm. Again, this thickness is sufficiently high to ensure that the wrapper 22 is robust enough to contain an absorbent article 10 and not tear easily. This thickness is sufficiently low enough to ensure that the wrapper 22 is flexible and can be easily folded. Thickness of a layer can be determine using any standard technics such as measuring the wrapper or paper layer with a caliper preferably a micrometer caliper gauge.

Depending on the dimensions of the cellulosic fibers, the layer consisting essentially of cellulosic fibers 33 can exhibit different behaviours. For example, using cellulosic fibers of higher fiber length improves the tear resistance whereas using cellulosic fibers of lesser fiber length improves the print quality and the surface smoothness of the wrapper 22. The paper layer 33 preferable comprises cellulosic fibers having an average fiber length comprised between 0,5 and 10 mm, preferably between 0,7 and 5 mm, more preferably between 1 and 3 mm. The cellulosic fibers preferably have an average fiber diameter comprised between 0,5 and 50 µm, preferably between 5 and 30 µm. The paper layer 33 have preferably a basis weight comprised between 10 gsm and 40 gsm.

According to an embodiment, the paper layer 33 comprises a first sub-layer 33a of cellulosic fibers and a second sub-layer 33b of cellulosic fibers. The first sub-layer 33a can be arranged on the garment-facing side thereby corresponding to the outer surface 25 of the wrapper. The second sub-layer 33b can be arranged between the first sub-layer 33a and the absorbent article 10. In this embodiment, it is preferable that the first sub-layer 33a have cellulosic fibers of different physical properties that the cellulosic fibers of the second sub-layer. For example the first sub-layer 33a can comprise cellulosic fibers with an average fiber length comprised between 0,5 mm and 3 mm, whereas the second sub-layer 33b comprises cellulosic fibers with an average fiber length comprised between 2 mm and 10 mm. In other words, the second sub-layer 33b has an average fiber length that is greater than the average fiber length of the first sub-layer 33a. This way the second sub-layer 33b ensures that the wrapper 22 exhibits a satisfying tear resistance whereas the first sub-layer 33a ensures a smooth surface with printing options on the pouch 22.

The layer consisting essentially of cellulosic fibers 33, whether comprising one layer or two sub-layers 33a,33b or more, can further comprise additives such as softeners, fillers and/or pigments, the latter to colour the paper.

Given that cellulosic fibers, in particular paper, is a material that can easily be printed on, the wrapper 22 according to the invention can be provided with graphic elements that are printed, by common techniques such as flexographic printing, gravure printing or inkjet printing, onto the outer surface 25 and/or inner surface 23 of the wrapper 22, in particular onto the layer consisting essentially of cellulosic fibers 33.

The absorbent article 10 comprises on the garment facing side of the backsheet 16 a layer of adhesive 28 so that the absorbent article 10 can adhere onto the inner surface 23 of the wrapper 22. The adhesive 28 also enables the pealing of the absorbent article 10 from the wrapper 22 so that it can be placed on an undergarment, the layer of adhesive 28 ensuring that the absorbent article 10 stays in place on the undergarment. The adhesive layer 28 can be applied on the entire garment facing surface of the backsheet 16, or the adhesive layer 28 can be applied on a portion of the garment facing surface of the backsheet 16 such as illustrated in Fig.3 where the adhesive layer 28 is not as wide or as long as the backsheet 16.

The layer of cellulosic fibers 33 can impede the good peeling of the adhesive layer 28 and thus of the absorbent article 10. To that end, the wrapper 22 preferably comprises a layer of peeling agent 32 or undergoes a peeling treatment. The peeling treatment may be carried out using any suitable means known without any particular limitation. For example, the peeling treatment can be carried out either by applying, or coating, peeling agent 32 material onto an area of the inner surface 23 of the wrapper 22 or by adhering a peelable tape or a peelable paper, to an area of the inner surface 23 of the wrapper 22.

The peeling agent or the peeling treatment applied to the peelable tape or the peelable paper thereof are preferably selected from and not limited to silicon resin series, fluororesin series, octadecilisocyanate series or the like. It is particularly preferable that the peeling treatment is carried out by applying a coat of silicon resin series as the peeling agent material. The peeling agent 32 is dried by heating or by irradiating ultraviolet rays or the like so as to be polymerized, or by spraying it, to thereby form a thin coat or thin layer of peeling agent 32. Using a thin coat of silicone as the peeling agent is preferable since silicone corresponds to a material that has heat-resistant properties, thus more stable in industrial processes, as well as antimicrobial properties which is better to ensure the protection of the absorbent article from contamination prior to use. The silicone has other properties such as water repellent and is greaseproof. The wrapper 22 comprises a layer of peeling agent 32 comprising a basis weight comprised between 0,3 gsm and 3 gsm, preferably between 0,5 gsm and 1 gsm.

Although the closing tape 24 is arranged to maintain the pouch 22 in a closed configuration, the absorbent article 10 being folded, will have a natural tendency to revert to its unfolded configuration and the closing tape 24 is usually not sufficient to maintain the absorbent article 10 in a folded configuration and the wrapper 22 closed. To that end, the wrapper 22 preferably comprises a layer of sealing agent 34 material and/or undergoes a sealing treatment to obtain sealed portion 30. The sealing treatment may be carried out using any suitable means known without any particular limitation. For example, the sealing treatment can be carried out either by applying, or coating, a sealing agent on an area of the inner surface 23 of the wrapper 22, folding the wrapper 22 and absorbent article 10 and then applying temperature and/or pressure to this agent material to achieve the sealing. For example and not limited to, the sealing treatment can be carried out by thermo-sealing (applying heat to a heat-sealable material), ultrasonic sealing (applying ultrasounds, thus heat, to a heat-sealable material), gluing (applying an adhesive material, preferably weak such as a non-structural adhesive, and then applying pressure).

For these embodiments comprising a sealing treatment as described above, i.e. implying the use of a sealing agent such as an adhesive or a heat-sealable material, it is preferable that the layer of sealing agent 34 comprises a nonpetroleum derived material as to lower the environmental impact of the absorbent article. The layer of sealing agent 34 comprises for example and not limited to polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL). The layer of sealing agent 34 can comprise a polymer material such as a polyethylene material, e.g. as a low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid. The sealing agent preferably comes from a sustainable source, or bio-based or bioplastic, and is biodegradable and/or compostable. The layer of sealing agent 34 has a basis weight comprised between 1 gsm and 20 gsm, preferably between 3 gsm and 15 gsm, more preferably between 5 gsm and 10 gsm.

According to an embodiment, the wrapper 22 comprises a second layer of sealing agent 34 arranged onto the layer of cellulosic fibers 33 on the opposite side of the wrapper 22 where the first layer of sealing agent 34 is arranged. In other words, the wrapper comprises two layers of sealing agent 34 arranged on both side of the wrapper, *i.e.* the wrapper 22 comprises a layer of sealing agent 34 on the inner surface 23 and a second layer of sealing agent 34 on the outer surface 25. In this embodiment, when folding the absorbent article 10 and the wrapper 22, the layer of sealing agent 34 on the outer surface 25 can come into contact with the layer of sealing agent on the inner surface 23 thereby improving the sealing when applying heat and/or pressure.

The invention is not limited to these sealing techniques. For example, the wrapper, preferably the layer of cellulosic fibers 33, can undergo mechanical embossing where the side-edges of the wrapper 22 containing the absorbent article 10, pass between two pairs of embossing rollers each pair arranged at opposite side of the folded packaged article and thereby embossing and causing the entanglement of the cellulosic fibers on each side of the packaged article to also obtain sealed sides 30. It is also possible to combine both techniques, heatsealing treatment and mechanical embossing.

The layer(s) of cellulosic fiber 33, the layer of peeling agent 32 and optionally the layer of sealing agent 34 form a laminate.

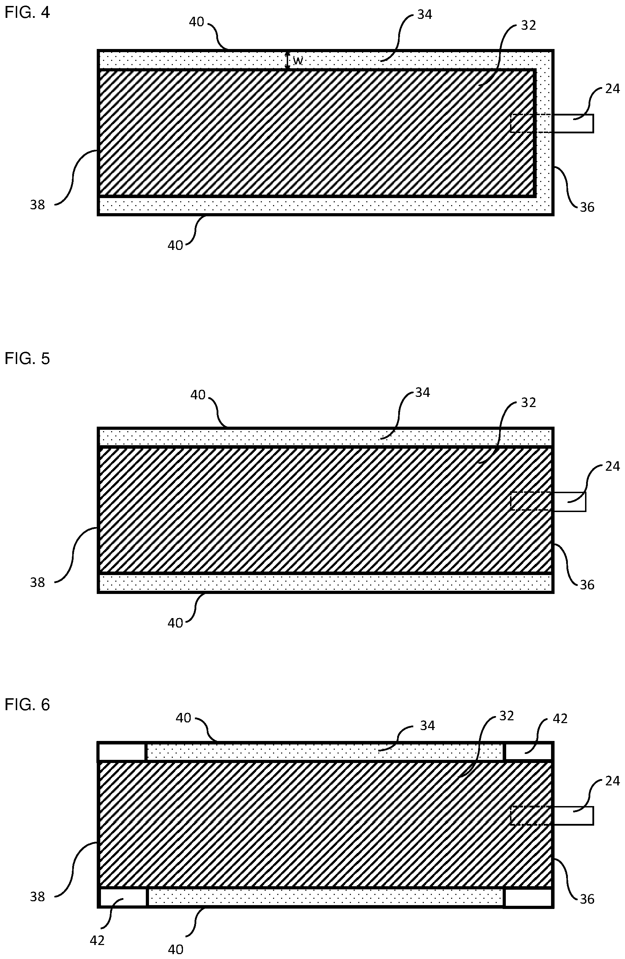

As illustrated in Fig. 4, the wrapper 22 has a generally rectangular configuration comprising a front end 36, a back end 38 arranged at the opposite side of the front end 36 and two side ends 40 arranged in-between. The front end 36, back end 38 and side ends 40 each having an edge, the side end 40 edges linking the front end 36 edge to the back end 38 edge. The wrapper 22 is provided at the front end 36 portion thereof with the closing tape 24 for sealing the wrapping construction.

The wrapper 22 of the present invention preferably has peelable properties where an area of the inner surface 23 of the wrapper 22 is subjected to peeling treatment. For example the area of the inner surface 23 of the wrapper 22 with which the adhesive layer 28 of the absorbent article is in contact and its vicinity can be subjected to peeling treatment. According to another embodiment, as illustrated here in Fig. 4, the majority of the area of the inner surface 23 is subjected to peeling treatment, more precisely, only the area excluding the peripheral edge portion of the inner surface 23 is subjected to peeling treatment. In other words, the wrapper 22 preferably comprises a layer of peeling agent 32, that layer, or coat, being arranged preferably on the inner surface 23 of the wrapper 22 at least on an area facing the adhesive layer 28 of the absorbent article 10 so as to ease the peeling of said absorbent article 10. The area of peeling agent 32 can be equal to or greater than the area defined by the adhesive layer 28. The adhesive layer 28 can be applied on a portion of the backsheet 16. The area of the peeling agent 32 can be arranged on the majority of the inner surface 23 of the wrapper 23. For example, the area of the peeling agent 32 coating is greater than 30%, preferably comprised between 40% and 95%, more preferably between 50% and 90% of the total area of the inner surface 23 of the wrapper 22.

The wrapper 22 of the present invention preferably has sealing properties where an area of the inner surface 23 and/or outer surface 25 of the wrapper 22 is subjected to a sealing treatment. For example the peripheral region of the inner surface 23 of the wrapper 22 can be subjected to a sealing treatment. According to another embodiment, as illustrated here in Fig. 4, Fig.5 or Fig.6, the minority of the area of the inner surface 23 is subjected to sealing treatment, more precisely, only the area comprising the peripheral edge portion of the inner surface 23 is subjected to a sealing treatment. In other words, the wrapper 22 preferably comprises a layer of sealing agent 34, that layer, or coat, being arranged preferably on the inner surface 23 of the wrapper 22 at least along the side ends 40 edges as to enable the side seals 30. The layer of sealing agent 34 can be arranged along a portion of, or at least partially along the side edges (Fig.6), along the entire length of the wrapper 22 along the side ends 40 edges (Fig.5) or along the entire length of the wrapper 22 along the side ends 40 edges and along the front end 36 edge (Fig.4). Of course the layer of sealing agent 34 can be arranged on the entire periphery of the wrapper 22. The coat of sealing agent 32 can be applied along an edge of the wrapper with a certain width w (as illustrated in Fig.4), w is comprised between 2 mm and 20 mm, preferably between 5 mm and 10 mm. Such dimensions enable a good sealing of the packaging and ensure that the absorbent article 10 is protected against contamination and maintaining a low environment impact. The width w refers to the distance on which sealing agent 34 is applied on the paper layer 33 relative to the side end 40 edges or relative to the front end 36 edge. The width w of sealing agent 34 at the side end 40 edges preferably have the same dimensions, e.g. between 5 mm and 10 mm, and can have the same or different dimension than the width w at the front end 36 edge. The cellulosic fibers forming the paper layer 33 are arranged in a continuous area, the sealing agent 34 is arranged in a discontinuous area (Fig. 5 or 6) or in a continuous area (Fig. 4) and the peeling agent 32 is also arranged in a continuous area.

The sealing agent 34 is preferably applied on the entire length of the wrapper along the edges of the side ends 40 (Fig.5). The sealing agent can also be applied on a portion of the length of the wrapper along the side edges 40 (Fig.6) where portions 42 of the wrapper 22 comprise the layer of cellulosic fibers 33 free of sealing agent 34 and free of peeling agent 32 coating. In other words, the layer of sealing agent 34 extends at least partially along the edges of the side ends 40. These coatless portions 42 are preferably arranged at least one of the corners of the rectangular configuration of the wrapper 22. These coatless portions 42 can for instance provide an additional grasping surface for the user to open up the packaged absorbent article 10.

As illustrated in the drawings, the wrapper 22 comprises a paper layer 33 on which is applied a coating of sealing agent 34 and a coating of peeling agent 32. The layer of sealing agent 34 is arranged on the periphery of the wrapper 22, along some of the edges, preferably along the two side ends 40 edges defining an opened frame with two panels defining an inner area. The peeling agent is arranged within the inner area of the frame defined by the sealing agent. In other words, the layer of peeling agent 32 is arranged at a central region of the inner surface 23 of the wrapper 22 whereas the layer of sealing agent 34 is arranged at a peripheral region of the inner surface 23 of the wrapper 22, relative to the coating of peeling agent 32. Another way to say is that the sealing agent is arranged at least partially around the peeling agent, or that the peeling agent is at least partially surrounded by the sealing agent.

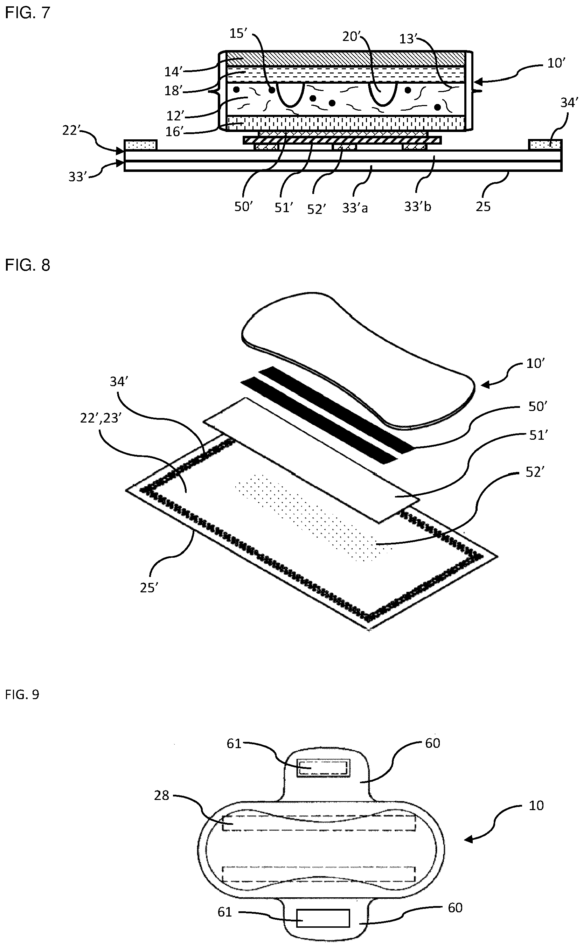

As illustrated in FIG. 7, a disposable absorbent article 10', for example a light incontinence pad, according to the present disclosure also comprises an absorbent core 12' arranged between a liquid permeable topsheet 14' and a liquid impermeable backsheet 16' similarly to the sanitary napkin. An optional acquisition distribution layer 18', can be positioned between said topsheet 14' and said absorbent core 12'. The absorbent core 12' comprises absorbent material selected from the group consisting of cellulose fibers 13' such as fluff pulp, superabsorbent polymers 15' or a combination thereof. The absorbent core 12' can comprise at least one channel 20' substantially free of cellulose fibers and/or substantially free of superabsorbent polymer (as illustrated in FIG. 7).

The backsheet 16' is joined to the topsheet 14' with the absorbent core 12', and the optional ADL 18', being positioned and held between the topsheet 14' and the backsheet 16'. As illustrated in FIG. 7, the topsheet 14' and the backseet 16' are associated to one another. The topsheet 14' is arranged on the body-facing side or surface whereas the backsheet 16' is arranged on the garment-facing side or surface.

For sake of conciseness, the rest of the description will focus on the differences between a sanitary napkin and a light incontinence pad.

The absorbent article 10' of the embodiment of FIG. 7 is also enclosed in a wrapper 22' or pouch, in a folded configuration prior to use, thereby forming an assembly according to the invention. The wrapper 22' acts as a barrier ensuring that the unused absorbent article 10' is protected against contamination prior to use. The wrapper 22' in light incontinence pads are seldom used as a disposal-wrap around a soiled absorbent article to be disposed of, hence there is no need for a closing tape.

Like sanitary napkins 10, light incontinence pads 10' also comprise a layer of adhesive 50', in order to provide the absorbent article 10' a fastening mean to the undergarment, namely by adhesive bonding. In order to protect the adhesive 28,50', the absorbent article 10,10' can either have a layer of peeling agent 32 on the wrapper 22 (FIG.1) or have a release paper 51' arranged between the layer of adhesive 50' and the wrapper 22' (FIG.7 or FIG. 8). In other terms, a light incontinence pad 10' usually comprises a release paper 51' arranged on the garment-facing side or surface of the backsheet 16', in particular on the garment-facing side or surface of the layer of adhesive 50'. The release paper 51' is preferably formed of a paper material such as woodfree paper or glassine paper, having a releasing layer, or releasing coating, of silicon-based resin. Naturally, the release paper 51' and the wrapper 22' can be formed from the same paper material to simplify the process and logistics. The release paper 51' preferably comprising a layer or coating of silicone-based resin, is easily peelable and can be easily separated from the absorbent article 10'. Such release paper offers more flexibility when applying the absorbent article and enables to lower the amount of silicone material used when comparing to a sanitary napkin that has is wrapped in a wrapper that is substantially covered by a peeling agent layer.

As illustrated in FIG. 7 and FIG. 8, the release paper 51' is arranged between the absorbent article 10' and the wrapper 22'. A first layer of adhesive 50' is arranged between the backsheet 16' of the absorbent article 10', more precisely, on the garment facing surface of the backsheet 16' and provides a releasable fixation means for the absorbent article 10' so that it can be secured to a garment. The first layer of adhesive 50' is illustrated here as comprising two elongated strips of adhesive material. Naturally, the first layer of adhesive 50' can comprise one larger elongated strip of adhesive material or the first layer of adhesive can comprise three, four, five or more strips of adhesive material. The strips can be arranged, or oriented, in the longitudinal direction of the absorbent article, meaning parallel to the longitudinal axis of the absorbent article as illustrated in FIG. 8, or alternatively, the first layer of adhesive can comprise a plurality of strips of adhesive material arranged, or oriented, in the transversal direction of the absorbent article, meaning orthogonal to the longitudinal axis of the absorbent article, in this case the first layer of adhesive 50' comprise more than 10 strips of adhesive material. When arranged in the transversal direction, it can be easier to peel off the absorbent article once used.

As illustrated in FIG. 8, the total surface area of the release paper 51' is greater than the total surface area of the first layer of adhesive 50', meaning that as seen from a top view, the area covered by the release paper 51' is greater than the total area occupied by the first layer of adhesive 50', thereby ensuring that the first adhesive layer 50' is well protected prior to use and the absorbent article 10' can be well secured to the undergarment. In other terms, the length and width of the release paper 51' is greater than the total length and width of the first adhesive layer 50'.

The assembly can optionally comprise a second layer of adhesive 52' that is arranged between the release paper 51' and the wrapper 22', meaning between the garment-facing side of the release paper 51' and the body-facing side of the wrapper 22', meaning the side of the wrapper facing the absorbent article 10'. The second layer of adhesive 52' can ensure that the absorbent article 10' stays attached to the wrapper 22' when opening said wrapper 22'.

The second layer of adhesive 52' is optional, as given that the absorbent article 10 and wrapper 22 are tri-folded, or folded in three, by unfolding only one end, the opposite unfolded end of the wrapper serves as a pocket, or recipient, for the absorbent article.

Preferably, the first adhesive layer 50' comprises a basis weight that is greater than the basis weight of the second adhesive layer 52'. Preferably, the first adhesive layer 50' comprises a basis weight that is at least 1.2 time greater than the basis weight of the second adhesive layer 52'. Naturally, the basis weight of the adhesive in the first adhesive layer 50' can be 1.3, 1.4, 1.5, 2, 3, 4, 5, 10, 20 or more times greater, or any values in between 1.2 and 20 times, than the basis weight of the second layer of adhesive 52'. For example, the second layer of adhesive 52' is arranged in a pattern with the adhesive material being applied in one or more swirls, dots, circles, triangles, rectangles or any other polygonal shape. The said adhesive material pattern can comprise for example a regular, irregular, staggered or linear application of adhesive material, meaning that the adhesive material can be applied for example in three discrete dots, in a regular checkerboard pattern, in two parallel swirled lines, *etc.* By having a lesser basis weight, when opening the wrapper 22' the absorbent article 10' stays attached to the wrapper 22' but it can be easily removed by the user.

Given that the first layer of adhesive 28,50' ensures that the absorbent article 10,10' stays fastened to the undergarment, the first layer of adhesive 28,50' comprises a basis weight between 10 gsm to 200 gsm, preferably between 15 gsm and 150 gsm depending on the type (sanitary napkin, light incontinence pad, pantiliner...), size, usage (day-time, night-time), *etc.* The first layer of adhesive 28,50' can comprise one elongated strip, or stripe, of adhesive, also called a block of adhesive, or two elongated stripes, or more. The first layer of adhesive 28,50' can also be applied in one or more swirls, dots, circles, triangles, rectangles or any polygonal adequate shape of adhesive material.

The adhesive layers 28,50',51' comprise an adhesive pattern. The adhesive pattern can comprise one, two or more elongated strips, or stripes, of adhesive material. The stripe(s) can be arranged in the longitudinal direction with a length by width ratio superior to 1 or the stripe(s) can be arranged in the transversal direction with a width by length ratio superior to 1, the length and width of the stripe(s) being the same as the length and width of the absorbent article. As mean of example, the adhesive pattern comprises two stripes of adhesive material each with a 55 mm x 15 mm (length x width) dimension with a 12 mm wide gap between.

This embodiment is not limited to this feature, for instance, the first and second layers of adhesive 50',52' can comprise the same amount of adhesive, but with different properties such as strength.

Alternatively, the second layer of adhesive 52' can ensure that the release paper 51' stays attached to the wrapper 22' when pulling out the absorbent article 10'. Given that, when present, the second layer of adhesive 52' is arranged between two layers of cellulosic fibers, meaning two layers comprising a material that adhere more to the adhesive than the silicone coating of the release paper, the amount of adhesive in the second layer of adhesive 52' can be increased so that when pulling on the absorbent article, the release paper 51' stays attached to the wrapper 22'. It is possible to have the same amount of adhesive in the first and second layers of adhesive 50',51' to ensure that the release paper 51' stays attached to the wrapper 22' when pulling on the absorbent article. In other words, the release paper 51' can be integrated in, or securely fastened to, the wrapper 22' in a similar manner as for a sanitary napkin 10 as described previously, while lowering the amount of silicone-based material used.

To summarize this optional feature, if the objective is to ensure that the release paper 51' stays attached to the absorbent article 10' when opening the wrapper 22', then the assembly either does not comprise any second adhesive layer or it comprises a second adhesive layer having a minute amount of adhesive, e.g. the first adhesive layer 50' comprises a basis weight that is at least five times or more greater than the basis weight of the second adhesive layer 52'. If the objective is to ensure that the release paper 51' stays attached to the wrapper 22' when opening the wrapper 22' then the assembly comprises a second adhesive layer having a lesser or equal amount of adhesive, e.g. the first adhesive layer 50' comprises a basis weight that is equal to, or 1.2 to 5 times greater than, the basis weight of the second adhesive layer 52', preferably 1.2 to 4 times greater than, more preferably 1.2 to 3 times greater than the basis weight of the second adhesive layer 52'.

The wrapper 22' can also comprise a structure as described previously for the sanitary napkin 10', while lowering the amount of silicone-based material used, where the wrapper 22' acts simultaneously as a release paper and a wrapping mean, or in other words, the release paper 51' is integrated in the wrapper 22'. In this case, if present, the second adhesive layer 52' can comprise a basis weight that is greater than the basis weight of the first adhesive layer 50'.

The amount of adhesive forming the first layer of adhesive 50' and second layer of adhesive 52' can be measured on a gram per square meter (gsm) basis. When measuring the amount of adhesive in the different layers based on a gram per square meter basis, the total weight of adhesive in grams in a particular adhesive layer can be divided by the surface or area (length x width) of said particular adhesive layer in the machine direction and in the cross direction.

For instance, the amount of adhesive in the first layer of adhesive 50' can be measured by taking a sample of the backsheet 16' (film or nonwoven fabric) where the first layer of adhesive 50' is arranged, weighing said sample before and after removal of the adhesive (in grams) the difference giving the weight of the adhesive, and dividing the weight of adhesive by the area of the sample (in m²) thereby obtaining the amount of adhesive in gsm. For example, the adhesive can be removed by using a solvent such as hexane. A similar analysis can be conducted in order to determine the amount of adhesive present in the second layer of adhesive 52' by taking a sample of the wrapper 22', weighing said sample before and after removal of the adhesive (in grams) the difference giving the weight of the adhesive, and dividing the weight of adhesive by the area of the sample (in m²) thereby obtaining the amount of adhesive in gsm. Naturally, the solvent used, such as hexane, mustn't affect the paper layer (wrapper) of the film or nonwoven (backsheet).

As shown in FIG. 8, the wrapper 22' has a generally rectangular configuration with an inner surface 23' on which the absorbent article 10' is arranged and an outer surface 25'. In other terms, the inner surface 23' is arranged on the body-facing side and the outer surface 25' is arranged on the garment-facing side.

Similarly as the sanitary napkin 10, the wrapper 22' for a light incontinence pad 10' also comprises a layer of sealing agent 34' as described previously, meaning with the same proprieties and/or materials. Said layer is arranged onto the layer of cellulosic fibers 33' preferably on the body-facing side of the wrapper 22'. The layer of sealing agent 34' is illustrated here as being arranged on the periphery of the wrapper 22' or alternatively, it can be arranged on the entire inner surface of the wrapper 22'. Naturally, the wrapper 22' can also comprise coatless portions as described previously.

The wrapper 22' for a light incontinent pad 10' can also have two layers of cellulosic fibers 33'a, 33'b as described previously. It is also possible to print graphic elements on the wrapper 22' for the light incontinence pad 10'.

The invention also relates to a process to manufacture an assembly as described above, the process comprising the following steps:
a) providing at least one layer of cellulosic fibers 33,33';
b) if the absorbent article is a sanitary napkin 10, applying a layer of peeling agent 32 on a surface of said at least one layer of cellulosic fibers 33;
c) optionally applying a layer of sealing agent 34 on a surface of said at least one layer of cellulosic fibers 33;
d) if the absorbent article is a sanitary napkin 10, applying an absorbent article 10 onto said layer of peeling agent in a way that the adhesive layer is arranged between the backsheet and the layer of peeling agent;
e) if the absorbent article is a light incontinence pad 10', applying an absorbent article 10' onto said layer of cellulosic fibers 33' or layer of sealing agent 34 in a way that the release paper 51' is arranged between the backsheet 16' and the wrapper 22';
f) folding the wrapper 22,22' and the absorbent article 10,10' altogether to enclose the absorbent article 10,10' within the wrapper; and
g) applying heat and/or pressure on the wrapper 22,22' to seal the absorbent article 10,10' within the wrapper 22,22';
h) optionally applying a closing tape on the outer surface of wrapper 22 namely if the absorbent article 10 is a sanitary napkin.

According to an embodiment, the process comprises an additional step of printing a graphic element on said wrapper 22,22'.

According to an embodiment, the process comprises an additional step of applying a second layer of adhesive 52' on the wrapper 22' prior to applying the light incontinence pad 10'.

It is supposed that the present invention is not restricted to any form of realization described previously and that some modifications can be added to the presented example of fabrication without reappraisal of the appended claims. As seen previously, both type of absorbent articles 10,10' comprise a wrapper 22,22' according to the invention, *i.e.* comprising a sustainable material such as a layer of cellulosic fibers and some features present in one type of absorbent article can be present in another type of absorbent article, unless specifically stated otherwise. For example, a sanitary napkin 10 can also comprise a release paper 51 as described hereabove. For instance, if a sanitary napkin 10, comprises wings 60 as illustrated in FIG. 9, a release paper 51 can be used to keep the wings 60 closed or folded when the absorbent article 10 is packed, the wings 60 each comprising a strip of adhesive 61 that are linked and maintained closed by a release paper 51. The layer of adhesive 28 here comprises two elongated stripes of adhesive material extending in the longitudinal direction of the absorbent article.

## Claims

1. Wrapper (22,22') for an absorbent article (10, 10') comprising a release paper (51,51'), said wrapper comprising a layer of cellulosic fibers (33,33') wherein the layer of cellulosic fibers (33,33') comprises a basis weight comprised between 10 gsm and 40 gsm.

2. Wrapper (22,22') according to claim 1, wherein the layer of cellulosic fibers (33,33') consists essentially of paper material.

3. Wrapper (22,22') according to any of the claims 1 or 2, wherein the layer of cellulosic fibers (33,33') comprises a basis weight comprised between 15 gsm and 35 gsm, more preferably between 16 gsm and 30 gsm, more preferably between 18 gsm and 29 gsm, more preferably between 20 gsm and 28 gsm.

4. Wrapper (22,22') according to any of the preceding claims, wherein the wrapper (22,22') further comprises a layer of sealing agent (34,34') arranged onto the layer of cellulosic fibers (33,33').

5. Wrapper (22,22') according to any of the preceding claims, wherein the layer of cellulosic fibers (33,33') comprises cellulosic fibers with an average fiber length comprised between 0,5 and 10 mm, preferably between 0,7 and 5 mm, more preferably between 1 and 4 mm and/or with an average fiber diameter comprised between 0,5 and 50 µm.

6. Wrapper (22,22') according to any of the preceding claims, wherein the layer of cellulosic fibers (33,33') comprises a thickness comprised between 5 µm and 150 µm, preferably between 20 µm and 80 µm.

7. Wrapper (22,22') according to any of the preceding claims, wherein the layer of cellulosic fibers (33,33') comprises a first sub-layer of cellulosic fibers (33a,33'a) and a second layer of cellulosic fibers (33b,33b'), the cellulosic fibers in the second sub-layer (33b,33'b) having a greater average fiber length than the first sub-layer (33a,33'a).

8. Wrapper (22,22') according to any of the preceding claims, wherein said wrapper further comprises a layer of peeling agent (32,32') comprising a peeling agent selected from silicon resin series, fluororesin series, octadecilisocyanate series, said layer of peeling agent (32) comprising a basis weight comprised between 0,3 gsm and 3 gsm, preferably between 0,5 gsm and 1 gsm.

9. Wrapper (22,22') according to any of the claims 4 and following, wherein the layer of sealing agent (34,34') has a basis weight comprised between 1 gsm and 20 gsm, preferably between 3 gsm and 15 gsm, more preferably between 5 gsm and 10 gsm.

10. Wrapper (22,22') according to any of the claims 4 and following, wherein the wrapper comprises a rectangular configuration, the sealing agent (34,34') being arranged at least on the periphery of the wrapper (22,22') along at least two edges arranged on opposite sides wherein the wrapper (22,22') further comprising uncoated portions (42,42') arranged at least at one of the corners of the rectangular configuration of the wrapper (22,22').

11. Assembly comprising a wrapper (22,22') according to any of the claims 1 to 10 and an absorbent article, the absorbent article (10,10') comprising a topsheet (14,14'), a backsheet (16,16') and an absorbent core (12,12') arranged in-between wherein the absorbent article (10,10') comprises a first adhesive layer (28,50') and a release paper (51'), preferably the first adhesive layer (50') is arranged in-between the backsheet (16') and the release paper (51').

12. Assembly according to claim 11, wherein the absorbent article (10,10') comprises wings (60) with release paper (51).

13. Assembly according to any of the claims 11 or 12, wherein the absorbent article (10') comprises a second adhesive layer (52') arranged between the wrapper (22') and the release paper (51').

14. Assembly according to claim 13, wherein the first adhesive layer (50') comprises a basis weight that is greater than the basis weight of the second adhesive layer (52'), preferably at least 1.2 time greater, more preferably at least 5 times greater.

15. Process to manufacture an assembly according to claim 11 to 14, the process comprising the following steps:
a. providing at least one layer of cellulosic fibers (33,33');
b. optionally applying a layer of sealing agent (34') on a surface of said at least one layer of cellulosic fibers (33',33'a,33'b);
c. applying an absorbent article (10') onto said layer of cellulosic fibers (33',33'a,33'b) or layer of sealing agent (34) in a way that the release paper (51') is arranged between the backsheet (16') and the wrapper (22');
d. folding the wrapper (22') and the absorbent article (10') altogether to enclose the absorbent article (10') within the wrapper; and
e. applying heat and/or pressure on the wrapper (22') to seal the absorbent article (10') within the wrapper (22').
